# EUROPEAN PATENT SPECIFICATION

(11) **EP 2 859 896 B1**
(45) Date of publication and mention of the grant of the patent: **31.01.2018**
(21) Application number: 13187712.8
(22) Date of filing: 08.10.2013
(51) Int. Cl.: A61K 31/7048, A61K 31/12, A61K 31/122, A61K 31/202, A61K 31/205, A61K 31/221, A61K 31/352, A61K 31/353, A61K 31/355, A61K 31/375, A61K 36/82, A61K 36/539, A61P 21/00

(54) **Pharmaceutical compositions for the treatment of muscular disorders**
Pharmazeutische Zusammensetzungen zur Behandlung von Muskelkrankheiten
Compositions pharmaceutiques pour le traitement des troubles musculaires

(43) Date of publication of application: 15.04.2015
(73) Proprietor: Ystem S.r.l., 20129 Milano (IT); U.G.A. Nutraceuticals S.r.l. unipersonale, 20900 Monza (MB) (IT)
(72) Inventor: Torrente, Yvan, 20129 Milano (IT); Selimi, Gentian, 20900 Monza (MB) (IT); Fabrizi, Francesco, 20900 Monza (MB) (IT)
(74) Representative: Barchielli, Giovanna

(56) References cited:
- WO-A1-2009/097512
- US-A1- 2004 109 906
- US-A1- 2008 233 245
- US-A1- 2013 210 753
- US-B2- 6 740 343
- THALOOR D ET AL: "Systemic administration of the NF-kappaB inhibitor curcumin stimulates muscle regeneration after traumatic injury.", THE AMERICAN JOURNAL OF PHYSIOLOGY AUG 1999, vol. 277, no. 2 Pt 1, August 1999 (1999-08), pages C320-C329, XP055102457, ISSN: 0002-9513
- POTGIETER M ET AL: "Histological assessment of SJL/J mice treated with the antioxidants coenzyme Q10 and resveratrol.", MICRON (OXFORD, ENGLAND : 1993) APR 2011, vol. 42, no. 3, April 2011 (2011-04), pages 275-282, XP055102441, ISSN: 1878-4291, DOI: 10.1016/j.micron.2010.10.001
- DATABASE WPI Week 201231 Thomson Scientific, London, GB; AN 2011-P81831 XP002720390, & CN 102 228 418 A (YUNNAN LONGRUN TEA GROUP CO LTD) 2 November 2011 (2011-11-02)

## Description

The present invention relates to pharmaceutical or nutritional compositions comprising baicalin and antioxidants and to their use in the treatment and/or prevention of muscular disorders or for improving muscle function.

### Background of the invention

There are many problems that can affect muscles. Muscle disorders can cause weakness, pain or even paralysis.

Causes of muscle disorders include injury or overuse, such as sprains or strains, cramps or tendinitis, genetic disorders, such as muscular dystrophies, some cancers, inflammation, such as myositis, diseases of nerves that affect muscles, infections, certain medicines. Sometimes the cause is not known.

Muscular dystrophies are the most severe case of muscle problems. Muscular dystrophy is a term that refers to a group of muscle disorders in which the face, arm, leg, spine, or heart muscles gradually shrink and weaken over time.

There are a variety of types of muscular dystrophy, including for example: Duchenne muscular dystrophy, facioscapulohumeral muscular dystrophy, the limb-girdle muscular dystrophies and mitochondrial myopathies.

The different types are distinguished by factors such as the age at which symptoms usually start, pattern of muscle weakness, speed at which the disease progresses, involvement of other tissues besides muscle and pattern of inheritance.

The symptoms and age of onset depend on the type of muscular dystrophy. Symptoms of muscular dystrophy often include: problems with coordination and mobility with frequent falls, muscle weakness and joint stiffness.

Many patients with muscular dystrophy show signs of inflammation; however, the mechanisms governing this inflammation in disease pathogenesis remain unexplored. Different in vivo studies showed that variations in mice diet, in particular feeding mdx mice with natural-derived antioxidants (green tea extract) (Buetler et al. 2002) or with a low iron diet, which reduces hydroxyl radicals (Bornman et al. 1998), could reduce signs of muscular damage. Similarly, artificial antioxidants such as IRFI-042, a synthetic vitamin E analogue, were proven to have strong antioxidant properties. Messina et al. showed that IRFI-042 reduces the activation of the transcription nuclear factor-κB (NF-κB), which can be activated by ROS, improving mdx muscle function. (Messina 2006).

Lonnrot et al. determined that CoQ10, a potent antioxidant, reduces the excessive oxidative radicals and calcium overload in Duchenne Muscular Dystrophy (DMD) muscles (Lonnrot K, Biochem Mol Biol Int 1998;44:727-737).

More recently, Gervasio et al demonstrated that treatment with L-Acetylcysteine ameliorates skeletal muscle pathophysiology in mdx mice (Gervasio (2008), 2003). Following these promising evidences, several clinical trials started using antioxidants in DMD patients. However, the results were disappointing due to a number of factors, which could account for the negative outcome (Rando, 2002).

Different treatment approaches were used so far trying to delay the progression of the disease but, unfortunately, it remains fatal. In spite of every effort, the only pharmacological treatment available are corticosteroids [hyser cl, 1988,429]:

Similarly to DMD, in dysferlinopathies the loss of dysferlin causes a defect in the membrane repair process which increases cellular susceptibility to damage.

Although many works reported beneficial effects of feeding dystrophic animal with a diet enriched in antioxidants, very little is known about using antioxidants in dysferlinopathies (Potgieter M. 2010).

US2013/210753 discloses in example 1 the use of flavocoxid (a mixture of cathechin and baicalin) in a model of muscular dystrophy (mdx mice). The disclosed composition does not contain further components, in particular DHA and EPA.

Nutritional supplements are used to enhance endurance capacity and muscle recovery, to increase muscle mass, to prevent muscle loss, to reduce muscle fatigue, to maintain muscle performance, muscle strength, and/or muscle function both in exercising and non-exercising individuals. For example, muscle mass and strength are lost during the aging process and mitigating such losses is an important part of a healthy aging process. Supplements can help to build muscle mass and strength that are lost over time and/or mitigate future losses. The most common supplements currently used are mixtures containing carbohydrates, creatine, proteins, dietary essential and non-essential amino acids, vitamins and/or minerals.

Despite advancements in the science of nutrition, simpler and more effective means of enhancing muscle mass and strength, physical performance and recovery from exercise with minimal undesirable side effects are needed.

### Description of the invention

We have now found that pharmaceutical or nutritional compositions containing baicalin and antioxidants and, in particular, pharmaceutical or nutritional compositions containing baicalin and curcumin, green tea catechins, vitamin C, coenzyme Q10, Acetyl-L-carnitine, vitamin E, DHA, EPA are useful in the treatment and/or prevention of muscular disorders or for improving muscle function.

Baicalin, which is a main active ingredient originally isolated from the root of Huangqin (*Scutellaria baicalensis* Georgi), has been used as an anti-inflammatory drug in traditional Chinese medicine. Previous studies have shown that baicalin could inhibit the proliferation of mononuclear cells, inhibit macrophage activation, inhibit the production of TH1 related cytokines in different murine models of disease. Burnett et al. (Journal of Medicinal Food 2007 Sep;10(3):442e51) suggested that baicalin acts via "dual inhibition" of COX and LOX enzymes to reduce the production of proinflammatory eicosanoids and attenuates edema in an in vivo model of inflammation. Liu et al. (Lishizhen Medicine and Materia Medica Research 2006;17(12):2377e9 found that early treatment of baicalin can lower the level of serum TNF-a and IL-6 in diet induced hyperlipidemic C57BL/6J mice.

Curcumin is the main curcuminoid of the popular Indian spice turmeric, which is a member of the ginger family (Zingiberaceae). The other two curcuminoids are desmethoxycurcumin and bis-desmethoxycurcumin. The curcuminoids are polyphenols and are responsible for the yellow color of turmeric. Curcumin can exist in at least two tautomeric forms, keto and enol. The enol form is energetically more stable in the solid phase and in solution. More recently, evidence that curcumin may have anti-inflammatory and anticancer activities has renewed scientific interest in its potential to prevent and treat diseases.

Zhu et al. (46th Annual Meeting American Society for Cell Biology - San Diego, December 2006) found that curcumin was significantly capable of attenuating dystrophic pathology and the mechanism underlying was involved in NF-κB inhibition.

Carnitine, derived from an amino acid, is nearly found in all cells of the body. Carnitine is the generic term for a number of compounds that include L-carnitine, acetyl-L-carnitine, and propionyl-L-carnitine. Carnitine plays a critical role in energy production. It transports long-chain fatty acids into the mitochondria so they can be oxidized ("burned") to produce energy. It also transports the toxic compounds generated out of this cellular organelle to prevent their accumulation. Given these key functions, carnitine is concentrated in tissues like skeletal and cardiac muscle that utilize fatty acids as a dietary fuel. The body makes sufficient carnitine to meet the needs of most people. For genetic or medical reasons, some individuals (such as preterm infants), cannot produce enough carnitine, so for them carnitine is a conditionally essential nutrient. Only L-carnitine is active in the body and is the form found in food. Carnitine has been proposed as a treatment for many conditions because it acts as an antioxidant.

Ascorbic acid, also known as Vitamin C, is a water-soluble vitamin necessary for normal growth, development and repair of damaged tissues in the body. It is essentially an antioxidant which is needed for the prevention of some of the damage occurring when one is exposed to cigarette smoke, radiation or when the body breaks down food. Ascorbic acid is primarily contained in fruits and vegetables, and can be naturally produced by some animals as it is derived from glucose. However, humans and a number of other vertebrates lack the ability to produce it and, therefore, required it as a dietary supplement, ideally on a daily basis in order to avoid symptoms of its deficiency which include, but are not limited to, inflammation of the gums, decreased resistance to disease-causing agents, scurvy and high blood pressure. It is needed for the growth and repair of worn-out and damaged tissues all over the body. It heals internal and external wounds and forms scar tissue by producing an essential protein which is used to make ligaments, blood vessels, tendons and skin. This vitamin also plays an important role in the repair and maintenance of bones, teeth and cartilages.

Coenzyme Q10, also referred to as CoQ10 or ubiquinone, is a vitamin-like compound which is present in all cells. It naturally occurs in the body and is found in highest amounts in the mitochondria, where cellular energy is created. CoQ10 levels are highest in the hardest-working tissues of the body, especially the heart. Coenzyme Q10 is an antioxidant, that is, it helps to protect cells from damage caused by the body's own free radicals.

Polyphenols have recently attracted attention because of their physiologic activity. Green tea, long consumed in Asian countries (mainly Japan and China), contains low-molecular-weight polyphenols mainly consisting of flavanol (flavan-3-ol) monomers, which are referred to as catechins. There are several isomers of this compound: catechin, catechin gallate (Cg), gallocatechin, gallocatechin gallate (GCg), epicatechin, epicatechin gallate (ECg), epigallocatechin, and epigallocatechin gallate (EGCg). Normally, 10-20% of the catechins in green tea leaves are epigallocatechin and EGCg. A portion of ingested EGCg is absorbed and widely distributed throughout the body. The ingestion of tea extract or catechins induces antioxidant, antiviral, antiplaque-forming and anticancer activities, it decreases blood pressure and blood sugar as well.

Omega-3 fatty acids, which are primarily found in fish oils but are also present in vegetable oils, are essential fatty acids - in that they are not produced by the body and must be supplied by the diet or supplements. Fish oil contains two types of omega-3 fatty acids: DHA (docosahexaenoic acid) and EPA (eicosapentaenoic acid). The majority of fish oil supplements contain 18% EPA and 12% DHA.

High-intensity exercise significantly increases the demand on the circulatory system to provide oxygen and nutrients to the muscles, lungs and brain, and to remove metabolic waste like lactic acid. Omega-3 essential fatty acids from fish oil have been shown to support lung function and to promote blood flow and oxygen delivery to active muscle by enhancing blood vessel function. They have also been shown to play an important part in recovery from physical stress. These effects combine to enhance endurance and promote joint flexibility, mobility and comfort.

Vitamin E refers to a group of eight fat-soluble compounds that include both tocopherols and tocotrienols. γ-Tocopherol can be found in corn oil, soybean oil, margarine and dressings. α-Tocopherol, the most biologically active form of vitamin E, can be most abundantly found in wheat germ oil, sunflower, and safflower oils. As a fat-soluble antioxidant, it stops the production of reactive oxygen species formed when fat undergoes oxidation.

The present invention relates to pharmaceutical or nutritional composition comprising baicalin, preferably in an amount of between 2 and 10% of the total weight of dry composition, and at least one antioxidant for use in the treatment and/or prevention of muscular disorders or for improving muscle function.

In particular the antioxidant is selected from the group consisting of vitamin E, coenzyme Q10, Acetyl-L-carnitine.

Object of the invention is pharmaceutical or nutritional compositions comprising:
a. baicalin;
b. curcumin;
c. green tea catechins;
d. vitamin E;
e. vitamin C;
f. coenzyme Q10;
g. Acetyl-L-carnitine;
h. DHA;
i. EPA;

Preferably, the compositions of the invention comprise:
a. baicalin in an amount of approx. between 30 mg and 500 mg;
b. curcumin in an amount of approx. between 500 mg and 1.500 mg;
c. green tea catechins in an amount of approx. between 30 mg and 200 mg;
d. vitamin E in an amount of approx. between 10 mg and 100 mg;
e. vitamin C in an amount of approx. between 100 mg and 500 mg;
f. coenzyme Q10 in an amount of approx. between 50 mg and 400 mg;
g. Acetyl-L-carnitine in an amount of approx. between 100 mg and 1000 mg;
h. DHA in an amount of approx. between 500 mg and 2000 mg;
i. EPA in an amount of approx. between 100 mg and 500 mg.

The pharmaceutical or nutritional compositions of the invention are useful in the treatment and/or prevention of muscular disorders. In particular, the treatment and/or prevention of muscular disorders include muscle atrophy, muscle wasting and associated disorders such as sarcopenia, cachexia, muscular damage, muscular dystrophies and muscular fatigue.

In particular, the compositions of the invention can be used in the treatment of muscular dystrophies such as Duchenne Muscular Dystrophy (DMD), facioscapulohumeral muscular dystrophy, limb-girdle muscular dystrophies and mitochondrial myopathies.

The compositions of the invention reduce stiffness and aches after a bout of exercise, resistance training or endurance training. Furthermore, they can be used in accelerating muscle recovery after muscle atrophy caused by immobilization, for example after bed rest and/or by plastering.

The compositions of the invention can be administered to mammals such as a human, a pet animal or a farm animal. The compositions can be administered daily during a period of at least 14 consecutive days and they can be administered orally, enterally or parenterally.

The compositions can be in the form of food compositions, dietary supplements, nutritional compositions, nutraceuticals, powdered nutritional products to be reconstituted in water or milk before consumption, food additives, medicaments, drinks and pet food or water. Preferably, the composition is a solid composition for reconstitution with any orally ingestible liquid or solid.

When the composition is dietary composition it may be in form of food such as dairy products (e.g. yoghurts), in form of fortified food such as cereal bars and bakery items such as cakes and cookies, in form of dietary supplements such as tablets, pills, granules, dragées, capsules and effervescent formulations, in form of non-alcoholic drinks such as soft drinks, sport drinks, fruit juices, lemonades, teas and milk based drinks, in form of liquid food such as soups and dairy products (muesli drinks).

The compositions of the invention ameliorated muscular force in mouse model and modulated their inflammation background, using a non-invasive procedure.

### EXAMPLE 1

### Formulation:

The formulation consists of an oily phase A and a powdered phase B:

### A: Oily Phase

Units: stick packs in single dose
Single unit dosage: 6.5 ml

| NUTRIENTS | | |
|---|---|---|
| Average contents | in 100 ml | in stick-pack (daily dose) |
| Docosahexaenoic acid DHA | 19.231 g | 1250.000 mg |
| Eicosapentaenoic acid EPA | 6.000 g | 360.000 mg |
| Vitamin E | 0.554 g | 36.000 mg |
| Flavouring | | |
| Essential oil of lemon fruits | 0.114 g | 7.395 mg |

Ingredients: Tuna Fish Oil 25% DHA,Vitamin E 67%, Essential oil of lemon fruits (C*itrus limonum*)*.*

### B: Powdered Phase

Units: Bag stick pack in single dose
Single unit weight: 5 g

| HERBAL COMPONENTS | | |
|---|---|---|
| Average contents | in 100g | in stick-pack (daily dose) |
| Curcuma phospholipid | 20.000 g | 1000.000 mg |
| Acetyl-L-carnitine | 15.000 g | 750.000 mg |
| Ascorbic acid | 4.800 g | 240.000 mg |
| Coenzyme Q10 | 4.000 g | 200.000 mg |
| Dried extract of Scutellaria root | 2.106 g | 105.300 mg |
| Dried extract of Green Tea leafs | 2.000 g | 100.000 mg |

| Excipients | |
|---|---|
| Average contents | in stick-pack (daily dose) |
| Fructose | 2212.200 mg |
| Sweetener | 300.000 mg |
| Silicon dioxide 50.000 mg | |
| Acesulfame K | 22.500 mg |
| Sucralose | 20.000 mg |
| Total | 5000.000 mg |

Ingredients: Fructose, curcuma phospholipid 20%, powdered Acetyl-L-carnitine HCl, flavouring, ascorbic acid, coenzyme Q10, Scutellaria (Scutellaria baicalensis Georgi) dried root extract 95% baicalin, green tea (Camellia Sinensis, maltodextrin, silica colloidal anhydrous) dried leafs extract 50% catechins, anti-caking agent: Silicon dioxide, sweetener: acesulfame K e sucralose.

### EXAMPLE 2

### Pharmacological results

### Force measurement

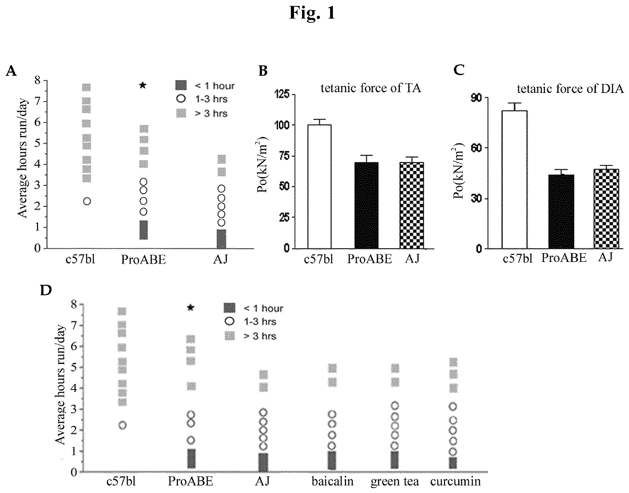

We treated two groups of animals: wild-type C57bl mice and dystrophic AJ mice. Mice were treated with 2-5mg/day of powdered phase and around 3-5 µl/day of oily phase for one month. Since the muscle pathology progressed in AJ mice as a function of age and in particular from 5 month of age, we fed 5 month old AJ mice with different antioxidants. To verify if antioxidants diet could improve muscular functionality, tetanic force of DIA and TA muscle and the endurance of mice were measured. No significant difference in maximum tetanic force between treated and control mice (Fig. 1: B and C) was found but an increase in endurance test in some antioxidants-fed mice (p<0.05) was observed. The data show that the treatment with the formulation of example 1 (hereinafter referred to as Proabe) significantly increases the endurance ability of dystrophic mice (Fig. 1: A).

Single compounds of powdered phase (Baicalin, curcumin and green tea) did not determine an amelioration of the endurance ability if they are administered alone, only the complete formulation guaranteed the performance (Fig. 1: D).

### Muscular and vascular features of AJ mice

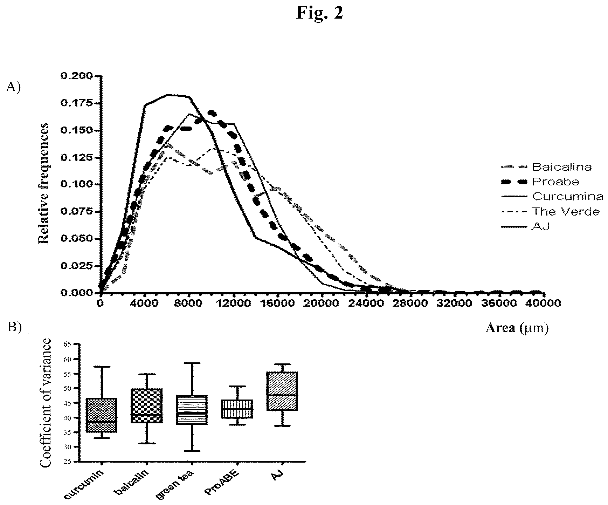

To verify whether this diet could delay the onset of the myopathy, a H&E analysis of muscle sections was performed. Treated mice showed the presence of degenerating and small centrally-nucleated regenerating muscle fibers, hypertrophic fibers, fiber splitting and fat replacement similar to AJ control mice. To investigate if antioxidant supplementation could have an effect on muscle mass, cross-section fiber area of treated and untreated mice was measured. The distribution curves of treated mice shift to the right if compared to that of the AJ control group, proving that there is a significant increase in fiber cross-section area (Mean: Baicalin 9677,39; Proabe 11711,75; Curcumin 9664,74; green tea 11214,04; AJ 8765,82) (Fig. 2: A). Moreover, the coefficient of variance of cross-section area was analyzed, confirming the results previously described (Fig. 2: B).

### Oxidative state evaluation

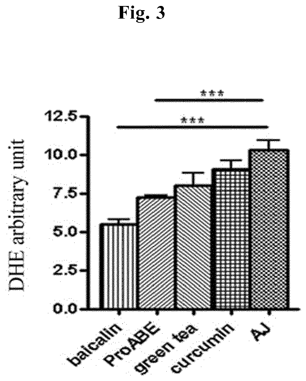

To evaluate whether polyphenol diet could influence ROS production, Dihydroethidium (DHE) staining in QA muscle from all mice was measured. As shown in the figure, (Fig. 3) DHE fluorescence intensity on muscle sections was measured. ProABE diet significantly reduced ROS production (p=0071**: 7.476 ± 0.6793).

### Vascular features

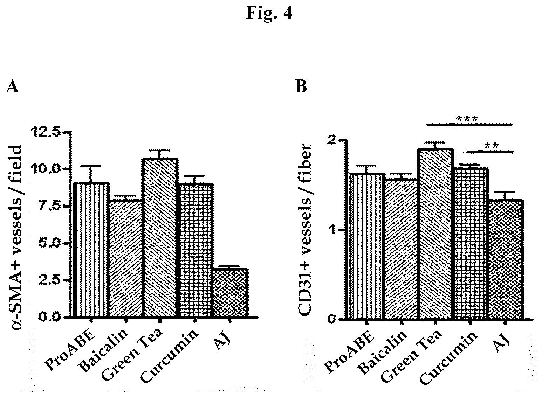

To test the influence of antioxidants on muscle vascular architecture, immunofluorescence staining on muscle sections was performed and the number of α-sma+ vessels was counted. All treated mice showed a significant increase in total α-sma vessels (p<0.001) demonstrating an amelioration of muscle perfusion without differences among the compounds used for treatments (Fig. 4: A). Therefore CD31+ vessels per fiber were counted. A partial reduction was found among all the antioxidants, but only mice fed with ProABE resulted to have an increase of CD31+ vessels per fiber (Fig. 4: B).

## Claims

1. A pharmaceutical or nutritional composition comprising:
a. baicalin;
b. curcumin;
c. green tea catechins;
d. vitamin E;
e. vitamin C;
f. coenzyme Q10;
g. Acetyl-L-carnitine;
h. docosahexaenoic acid (DHA);
i. eicosapentaenoic acid (EPA).

2. The pharmaceutical or nutritional composition according to claim 1 comprising:
a. baicalin in an amount of approx. between 30 mg and 500 mg;
b. curcumin in an amount of approx. between 500 mg and 1.500 mg;
c. green tea catechins in an amount of approx. between 30 mg and 200 mg;
d. vitamin E in an amount of approx. between 10 and 100 mg;
e. vitamin C in an amount of approx. between 100 mg and 500 mg;
f. coenzyme Q10 in an amount of approx. between 50 mg and 400 mg;
g. Acetyl-L-carnitine in an amount of approx. between 100 mg and 1000 mg;
h. docosahexaenoic acid (DHA) in an amount of approx. between 500 mg and 2000 mg;
i. eicosapentaenoic acid (EPA) in an amount of approx. between 100 mg and 500 mg.

3. The pharmaceutical or nutritional composition according to claim 1 or 2 for use in the treatment and/or prevention of muscular disorders.

4. The pharmaceutical or nutritional composition for the use according to claim 3 wherein the use in the treatment and/or prevention of muscular disorders includes muscle atrophy, muscle wasting and associated disorders such as sarcopenia, cachexia, muscular damage, muscular dystrophies and muscular fatigue or for improving muscle function and endurance, for preventing muscle loss, for enhancing muscle recovery, for reducing muscle fatigue.

5. The pharmaceutical or nutritional composition according to claim 1 or 2 for oral, enteral or parenteral administration.

6. The pharmaceutical or nutritional composition according to claim 1 or 2 wherein the composition is selected from the group consisting of food compositions, dietary supplements, nutritional compositions, nutraceuticals, powdered nutritional products to be reconstituted in water or milk before consumption, food additives, medicaments, drinks and pet food or water.

7. The pharmaceutical or nutritional composition according to claim 1 or 2 wherein the composition is a dietary composition in form of food, fortified food, bakery products, dietary supplements in form of tablets, pills, granules, dragées, capsules and effervescent formulations, non-alcoholic drinks, sport drinks, fruit juices, lemonades, teas and milk based drinks, liquid food, dairy products.

## Patentansprüche

1. Pharmazeutische oder Nährstoff-Zusammensetzung, umfassend:
a. Baicalin;
b. Curcumin;
c. Grünteekatechine;
d. Vitamin E;
e. Vitamin C;
f. Coenzym Q10;
g. Acetyl-L-carnitin;
h. Docosahexaensäure (DHA);
i. Eicosapentaensäure (EPA).

2. Pharmazeutische oder Nährstoff-Zusammensetzung gemäß Anspruch 1, umfassend:
a. Baicalin in einer Menge von zwischen etwa 30 mg und 500 mg;
b. Curcumin in einer Menge von zwischen etwa 500 mg und 1.500 mg;
c. Grünteekatechine in einer Menge von zwischen etwa 30 mg und 200 mg;
d. Vitamin E in einer Menge von zwischen etwa 10 bis 100 mg;
e. Vitamin C in einer Menge von zwischen etwa 100 mg und 500 mg;
f. Coenzym Q10 in einer Menge von zwischen etwa 50 mg und 400 mg;
g. Acetyl-L-carnitin in einer Menge von zwischen etwa 100 mg und 1.000 mg;
h. Docosahexaensäure (DHA) in einer Menge von zwischen etwa 500 mg und 2.000 mg;
i. Eicosapentaensäure (EPA) in einer Menge von zwischen etwa 100 mg und 500 mg.

3. Pharmazeutische oder Nährstoff-Zusammensetzung gemäß Anspruch 1 oder 2 zur Verwendung in der Behandlung und/oder Vorbeugung von Muskelerkrankungen.

4. Pharmazeutische oder Nährstoff-Zusammensetzung zur Verwendung gemäß Anspruch 3, wobei die Verwendung in der Behandlung und/oder Vorbeugung von Muskelerkrankungen Folgendes umfasst: Muskelatrophie, Muskelschwund und assoziierte Erkrankungen, wie zum Beispiel Sarkopenie, Kachexie, Muskelschädigung, Muskeldystrophien und Muskelermüdung oder zur Verbesserung der Muskelfunktion und -ausdauer, zur Vorbeugung von Muskelabbau, zur Verstärkung des Muskelaufbaus, zur Verringerung der Muskelermüdung.

5. Pharmazeutische oder Nährstoff-Zusammensetzung gemäß Anspruch 1 oder 2 zur oralen, enteralen oder parenteralen Verabreichung.

6. Pharmazeutische oder Nährstoff-Zusammensetzung gemäß Anspruch 1 oder 2, wobei die Zusammensetzung ausgewählt ist aus der Gruppe, bestehend aus Nahrungsmittelzusammensetzungen, Nahrungsergänzungsmitteln, Nährstoffzusammensetzungen, Nutraceuticals, pulverförmigen Nährstoffprodukten zur Rekonstitution in Wasser oder Milch vor dem Gebrauch, Nahrungsmittelzusätzen, Medikamenten, Getränken und Tiernahrung oder Wasser.

7. Pharmazeutische oder Nährstoff-Zusammensetzung gemäß Anspruch 1 oder 2, wobei die Zusammensetzung eine Diätzusammensetzung in Form von Nahrungsmitteln, nährstoffangereicherter Nahrung, Bäckereiprodukten, Nahrungsergänzungsmitteln in Form von Tabletten, Pillen, Granuli, Dragees, Kapseln und schäumenden Formulierungen, nichtalkoholischen Getränken, Sportgetränken, Fruchtsäften, Limonaden, Tees und milchbasierten Getränken, Flüssignahrung und Molkereiprodukten ist.

## Revendications

1. Composition pharmaceutique ou nutritionnelle comprenant :
a. de la baïcaline ;
b. de la curcumine ;
c. des catéchines de thé vert ;
d. de la vitamine E ;
e. de la vitamine C ;
f. de la coenzyme Q10 ;
g. de l'acétyl-L-carnitine ;
h. de l'acide docosahexaénoïque (DHA, « *Docosahexaenoic acid* ») ;
i. de l'acide eicosapentaénoïque (EPA, « *Eicosapentaenoic acid* »)*.*

2. Composition pharmaceutique ou nutritionnelle selon la revendication 1, comprenant :
a. de la baïcaline en une quantité approximativement entre 30 mg et 500 mg ;
b. de la curcumine en une quantité approximativement entre 500 mg et 1 500 mg ;
c. des catéchines de thé vert en une quantité approximativement entre 30 mg et 200 mg ;
d. de la vitamine E en une quantité approximativement entre 10 et 100 mg ;
e. de la vitamine C en une quantité approximativement entre 100 mg et 500 mg ;
f. de la coenzyme Q10 en une quantité approximativement entre 50 mg et 400 mg ;
g. de l'acétyl-L-carnitine en une quantité approximativement entre 100 mg et 1000 mg ;
h. de l'acide docosahexaénoïque (DHA, « *Docosahexaenoic acid* ») en une quantité approximativement entre 500 mg et 2000 mg ;
i. de l'acide eicosapentaénoïque (EPA, « *Eicosapentaenoic acid* ») en une quantité approximativement entre 100 mg et 500 mg.

3. Composition pharmaceutique ou nutritionnelle selon la revendication 1 ou 2 pour utilisation dans le traitement et/ou la prévention des troubles musculaires.

4. Composition pharmaceutique ou nutritionnelle pour l'utilisation selon la revendication 3, dans laquelle l'utilisation dans le traitement et/ou la prévention des troubles musculaires comprend l'atrophie musculaire, la fonte musculaire et des troubles associés tels que la sarcopénie, la cachexie, la lésion musculaire, les dystrophies musculaires et la fatigue musculaire ou pour améliorer la fonction musculaire et l'endurance, pour prévenir la perte des muscles, pour améliorer la récupération musculaire, pour réduire la fatigue musculaire.

5. Composition pharmaceutique ou nutritionnelle selon la revendication 1 ou 2 pour administration par voie orale, entérale ou parentérale.

6. Composition pharmaceutique ou nutritionnelle selon la revendication 1 ou 2, la composition étant choisie dans le groupe constitué par les compositions alimentaires, les compléments alimentaires, les compositions nutritionnelles, les produits nutraceutiques, les produits nutritionnels sous forme de poudre à reconstituer dans de l'eau ou du lait avant consommation, les additifs alimentaires, les médicaments, les boissons et la nourriture ou l'eau pour animaux de compagnie.

7. Composition pharmaceutique ou nutritionnelle selon la revendication 1 ou 2, la composition étant une composition alimentaire sous forme de nourriture, d'aliments enrichis, de produits de boulangerie, de compléments alimentaires sous forme de comprimés, de pilules, de granulés, de dragées, de capsules et de formulations effervescentes, de boissons non alcoolisées, de boissons pour sportifs, de jus de fruits, de limonades, de thés et de boissons à base de lait, d'aliments liquides, de produits laitiers.
